# EUROPEAN PATENT APPLICATION

(11) **EP 4 331 625 A1**
(43) Date of publication of application: **06.03.2024**
(21) Application number: 22795313.0
(22) Date of filing: 11.03.2022
(51) Int. Cl.: A61K 51/04, A61K 39/395, A61K 47/68, A61K 51/10

(54) **METHOD FOR STORING INTERMEDIATE FOR RADIOPHARMACEUTICAL COMPOSITION, METHOD FOR PREPARATION OR STORAGE OF RADIOPHARMACEUTICAL COMPOSITION, INTERMEDIATE COMPOSITION FOR RADIOPHARMACEUTICAL COMPOSITION, AND PHARMACEUTICAL FORMULATION**

(30) Priority: 27.04.2021 JP 2021075227
(71) Applicant: National Institutes for Quantum Science and Technology, Chiba 263-8555 (JP)
(72) Inventor: YOSHII, Yukie, Chiba-shi, Chiba 263-8555 (JP); MATSUMOTO, Hiroki, Chiba-shi, Chiba 263-8555 (JP); IGARASHI, Chika, Chiba-shi, Chiba 263-8555 (JP)
(74) Representative: Plasseraud IP
(86) International application number: PCT/JP2022/010771
(87) International publication number: WO 2022/230390

(57) **Abstract**

A storage method according to an aspect of the present invention includes storing an intermediate of a radiopharmaceutical composition in an acetate buffer. The intermediate is a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody). The intermediate is stored for a storage period of not less than 24 hours.

## Description

### Technical Field

The present invention relates to a storage method for an intermediate of a radiopharmaceutical composition, a preparation or storage method for the radiopharmaceutical composition, an intermediate composition of the radiopharmaceutical composition, and a pharmaceutical formulation.

### Background Art

Radioactive antibody drugs which are obtained by labeling with a radionuclide with use of a bifunctional chelator is under research and development. The bifunctional chelator is, for example, a 3,6,9,15-tetraazabicyclo[9.3.1]pentadeca-1(15), 11, 13-triene-3,6,9-triacetic acid (PCTA). For example, Non-Patent Literatures 1 to 5 each disclose a method for radioimmunotherapy and diagnosis with use of a radioactive antibody drug.

### Citation List

### [Non-patent Literature]

[Non-patent Literature 1] Yoshii et al., Sci Rep 2020; 10: 4143
[Non-patent Literature 2] Yoshii et al., J Nucl Med. 2019; 60: 1437-1443
[Non-patent Literature 3] Yoshii et al., Oncotarget. 2018. 9: 28935-28950
[Non-patent Literature 4] Song IH et al., Oncotarget. 2017; 8: 92090-92105
[Non-patent Literature 5] Song IH et al., J Nucl Med. 2016; 57: 1105-11

### Summary of Invention

### Technical Problem

However, with the above-described conventional technique, long-term storage stability of an antibody-ligand complex (antibody-chelator complex), which is an intermediate of a radioactive antibody drug, is not ensured. Accordingly, extemporaneous preparation of the radioactive antibody drug is essential. Further, in a case where extemporaneous preparation is performed, stable supply of the radioactive antibody drug is considered to be difficult. This is because, for example, aseptic manipulation is necessary, skill is needed for such an operation, and there is a risk of exposure to radiation. Further, when the radioactive antibody drug is to be extemporaneously prepared, only a limited volume of the radioactive antibody drug can be produced at a time. Accordingly, it is considered that production cost of the radioactive antibody drug is likely to increase. In light of the above, development of a technology for producing radioactive antibody drugs stably at low cost has been desired.

An object of an aspect of the present invention is to provide a technology for producing a radioactive antibody drug without performing extemporaneous preparation.

### Solution to Problem

As a result of making diligent studies, the inventors of the present invention have found that with use of a stock solution having a specific composition, an intermediate of a radioactive antibody drug can be stored for a long term. Further, the inventors have found that physical properties of a radioactive antibody drug which is prepared from an intermediate of the radioactive antibody having been stored in the stock solution for a certain period of time are equivalent to those of a radioactive antibody drug which is obtained by extemporaneous preparation. The inventors of the present invention have thus accomplished an aspect of the present invention.

A storage method in accordance with an aspect of the present invention is a storage method including storing an intermediate of a radiopharmaceutical composition in an acetate buffer, the intermediate being a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody), and the intermediate being stored for a storage period of not less than 24 hours.

Further, a storage method in accordance with an aspect of the present invention is a storage method, including storing the radiopharmaceutical composition in an acetate buffer, the radiopharmaceutical composition being labeled with a radionuclide which is coordinated with a 3,6,9,15-tetraazabicyclo[9.3.1]pentadeca-1(15),11,13-triene-3,6,9-triacetic acid (PCTA) and being a complex of the PCTA and an anti-epidermal growth factor receptor antibody (anti-EGFR antibody).

Further, an intermediate composition in accordance with an aspect of the present invention is an intermediate composition that is used for preparation of a radiopharmaceutical composition, the intermediate composition including an intermediate of the radiopharmaceutical composition and a stock solution, the intermediate being a complex of a 3,6,9,15-tetraazabicyclo[9.3. 1]pentadeca-1(15),11,13-triene-3,6,9-triacetic acid (PCTA) and an antibody, the stock solution containing at least one type of neutral amino acid, at least one type of polysorbate, and an acetate buffer, and the intermediate having been stored in the stock solution for not less than 24 hours.

Further, a pharmaceutical composition in accordance with an aspect of the present invention is a pharmaceutical formulation, including a radiopharmaceutical composition and a stock solution, the radiopharmaceutical composition being labeled with a radionuclide which is coordinated with a 3,6,9,15-tetraazabicyclo[9.3. 1]pentadeca-1(15),11,13-triene-3,6,9-triacetic acid (PCTA), the radionuclide being a complex of the PCTA and an antibody, the stock solution containing at least one type of neutral amino acid, at least one type of polysorbate, and an acetate buffer, and the radiopharmaceutical composition being obtained by labeling, with the radionuclide that is coordinated with the PCTA, the complex which has been stored in the stock solution for not less than 24 hours.

### Advantageous Effects of Invention

According to an object of an aspect of the present invention, it is possible to produce a radioactive antibody drug without performing extemporaneous preparation.

### Brief Description of Drawings

Fig. 1 is a diagram showing an effect of a stock solution on a labeling yield of ⁶⁴Cu-PCTA-cetuximab.
Fig. 2 is a diagram showing an effect of a stock solution on stability of ⁶⁴Cu-PCTA-cetuximab after labeling in a case where PCTA-cetuximab is stored in the stock solution for three months.
Fig. 3 is a diagram showing an effect of a stock solution on stability of ⁶⁴Cu-PCTA-cetuximab after labeling in a case where PCTA-cetuximab is stored in the stock solution for 12 months.
Fig. 4 is a diagram showing an effect of the stock solution on cell-binding properties of ⁶⁴Cu-PCTA-cetuximab.
Fig. 5 is a diagram showing how radioactivity at the time of labeling influences the cell-binding properties of ⁶⁴Cu-PCTA-cetuximab.
Fig. 6 is a diagram showing an effect of the stock solution with respect to stability of ²²⁵Ac-PCTA-cetuximab after labeling.

### Description of Embodiments

### [Storage method for intermediate of radiopharmaceutical composition]

A storage method in accordance with the present embodiment for an intermediate of a radiopharmaceutical composition (which may be, hereinafter, abbreviated as "the intermediate storage method in accordance with the present embodiment") includes storing, in a stock solution, an intermediate of a radiopharmaceutical composition.

### (Radiopharmaceutical composition and intermediate thereof)

In the present specification, the radiopharmaceutical composition refers to a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody). The complex is labeled with a radionuclide. The radiopharmaceutical composition can be used in diagnosis or treatment of a disease. Further, in the present specification, the intermediate of the radiopharmaceutical composition refers to a pharmaceutical composition prior to labeling with a radionuclide (that is, a complex of PCTA and anti-EGFR antibody, which is not labeled with the radionuclide). The radionuclide will be discussed later.

### (PCTA)

The PCTA is a bifunctional chelator. Into the PCTA, an isocyanate group or an N-hydroxysuccinimidyl group is introduced. Then, on a matter thus obtained, a side-chain amino group of a lysine residue or an N-terminal amino group of an antibody is caused to act, so that a chelating site is introduced into the antibody. Thereafter, at the chelating site, complex forming of the radionuclide is carried out, so that an antibody labeled with the radionuclide can be obtained.

### (Anti-EGFR antibody)

Examples of the anti-EGFR antibody include an antibody that specifically binds to cancer. Specific examples of the anti-EGFR antibody include cetuximab, panitumumab, and necitumumab. The anti-EGFR antibody may be a mouse antibody, a chimeric antibody, a humanized antibody, and a human antibody. Further, the anti-EGFR antibody may be a monoclonal antibody or a polyclonal antibody. The anti-EGFR antibody used can be a commercially available anti-EGFR antibody or an anti-EGFR antibody synthesized by a well-known method.

The intermediate used may be an intermediate synthesized by a well-known method. For example, as described in Examples, the intermediate can be obtained by: mixing a solution containing the PCTA and a solution containing the anti-EGFR antibody together; and incubating a resultant mixture at 1°C to 60°C for 1 hours to 24 hours.

### (Stock solution of intermediate)

The stock solution used in the storage method in accordance with the present embodiment for the intermediate (which, hereinafter, may be referred to as the "stock solution of the intermediate") is an acetate buffer. The acetate buffer preferably has a pH of 5 to 8. The use of the acetate buffer as the stock solution reduces protein aggregation and allows the intermediate to be stored for a desired period of time.

The concentration of the intermediate contained in the above stock solution may be not less than 0.1 mg/mL, and not less than 1 mg/mL. Further, the concentration may be not more than 50 mg/mL, and may be not more than 5 mg/mL.

It is preferable that the stock solution of the intermediate preferably contain a neutral amino acid, in terms of, for example, having radical-scavenging activity. Among neutral amino acids, a neutral amino acid having high solubility is more preferable, in terms, for example, increasing stability of the antibody in the stock solution. Examples of the neutral amino acid having high solubility include asparagine, glutamine, serine, threonine, cysteine, alanine and glycine. One type of the above neutral amino acids may be contained in the stock solution, or two or more types of the above neutral amino acid may be contained in the stock solution.

The concentration of the neutral amino acid contained in the stock solution of the intermediate is preferably not less than 1 pM, more preferably not less than 10 pM, and even more preferably not less than 50 pM, in terms of, for example, increasing the stability of the antibody in the stock solution. Further, the concentration of the neutral amino acid contained in the above stock solution is preferably not more than 260 mM, more preferably not more than 200 mM, and even more preferably not more than 150 mM.

The stock solution of the intermediate preferably contains polysorbate, because, for example, containing polysorbate allows the stock solution of the intermediate to have radical-scavenging activity and to stably store the intermediate by suppressing antibody (protein) aggregation. Examples of the polysorbate include polysorbate 20, polysorbate 40, polysorbate 60, polysorbate 65, and polysorbate 80. One type of the above polysorbates may be contained in the stock solution, or two or more types may be contained in the stock solution.

The concentration of the polysorbate contained in the stock solution of the intermediate is preferably not less than 1 pM, more preferably not less than 10 pM, and even more preferably not less than 50 pM, because at such a concentration, protein aggregation is suppressed and the intermediate can be stably stored. Further, the concentration of the polysorbate contained in the above stock solution is preferably not more than 400 mM, more preferably not more than 200 mM, and even more preferably not more than 100 mM.

It is more preferable that the stock solution of the intermediate contain at least one type of neutral amino acid and at least one type of polysorbate. In a case where the stock solution, which is an acetate buffer, contains a neutral amino acid and polysorbate, a synergistic effect of the acetate buffer, the neutral amino acid and the polysorbate can be exerted. Then, protein aggregation is further suppressed. As a result, the intermediate can be stably stored. This further provides the effect of suppressing radioactive degradation of the radiopharmaceutical composition prepared with use of the intermediate stored in the stock solution.

The intermediate is stored for a storage period of not less than 24 hours. The intermediate may be stored for a storage period of not less than 48 hours, not less than 1 week, or not less than 1 month. Further, the intermediate may be stored for a storage period of not more than 3 years, not more than 2 years, and not more than 12 months.

The storage temperature of the intermediate is preferably not less than 1°C, and more preferably not less than 3°C, in terms of stability etc. of the intermediate. Further, the storage temperature of the intermediate is preferably not more than 8°C, and more preferably not more than 5°C.

The storage method in accordance with the present embodiment for the intermediate may include another step(s). Examples of such a step includes the step of replacing, by the stock solution described above, a solvent of the intermediate after synthesis.

The present embodiment also encompasses an intermediate stored by the storage method in accordance with the present embodiment for the intermediate, and a stock solution (intermediate composition) containing the intermediate after storage by the storage method. Further, the present embodiment also encompasses a kit including the intermediate composition and a radionuclide for labeling the intermediate. As described in Examples, the radiopharmaceutical composition prepared by the intermediate stored by the storage method in accordance with the present embodiment for the intermediate has a radiochemical purity with which the intermediate can be used in real clinical settings.

In the conventional technique, since a long-term storage method for the intermediate was not established, extemporaneous preparation of the intermediate was necessary. However, a radiopharmaceutical composition prepared from an intermediate which has been stored for 12 months by the storage method in accordance with the present embodiment for the intermediate has physical properties equivalent to those of a radiopharmaceutical composition which has been obtained by extemporaneous preparation. Therefore, extemporaneous preparation of the intermediate becomes unnecessary by using the storage method in accordance with the present embodiment for the intermediate. This makes it possible to reduce cost of anti-EGFR antibody and PCTA, which are raw materials of the intermediate. Further, since extemporaneous preparation of the intermediate becomes unnecessary, personnel cost for preparation of the intermediate and quality testing of the intermediate can be significantly reduced. In addition, the storage method in accordance with the present embodiment for the intermediate can conform to the pharmaceutical good manufacturing practice (GMP).

### [Preparation method of radiopharmaceutical composition]

The preparation method in accordance with the present embodiment for a radiopharmaceutical composition includes a labeling step of labeling, with a radionuclide that is coordinated with PCTA, the above intermediate which has been stored by the above storage method.

### (Labeling step)

The radionuclide used in this labeling step is a radionuclide that is coordinated with PCTA. Examples of the radionuclide include ⁶⁴Cu and ²²⁵Ac.

The intermediate can be labeled with the radionuclide by a well-known method. For example, as shown in Examples, the stock solution containing the intermediate is mixed with a solution containing the radionuclide. Further, a resultant mixture is incubated at 1°C to 60°C for 5 minutes to 12 hours. This makes it possible to label the intermediate with the radionuclide. The solution containing the radionuclide is preferably an acetate buffer, and more preferably an acetate buffer having the same pH as the stock solution.

The preparation method in accordance with the present embodiment for the radiopharmaceutical composition may include another step in addition to the labeling step. Examples of such a step include the step of measuring the radiochemical purity of the radiopharmaceutical composition.

### [Storage method for radiopharmaceutical composition]

A storage method in accordance with the present embodiment for a radiopharmaceutical composition includes storing the radiopharmaceutical composition in a stock solution.

### (Stock solution of radiopharmaceutical composition)

The stock solution used by the storage method in accordance with the present embodiment for the radiopharmaceutical composition (which, hereinafter, may be referred to as "stock solution of the radiopharmaceutical composition"), like the stock solution of the intermediate, is an acetate buffer. The use of the acetate buffer as the stock solution reduces protein aggregation and allows the radiopharmaceutical composition to be stored for a desired period of time.

A preferable embodiment of the stock solution of the radiopharmaceutical composition is similar to that of the stock solution of the intermediate. In particular, containing a neutral amino acid and polysorbate in the stock solution of the radiopharmaceutical composition that is an acetate buffer further suppresses protein aggregation. As a result, the radiopharmaceutical composition can be stably stored. This further provides the effect of suppressing radioactive degradation of the radiopharmaceutical composition.

The stock solution of the radiopharmaceutical composition and the stock solution of the intermediate may be the same or different. It is preferable that the stock solution of the radiopharmaceutical composition and the stock solution of the intermediate be the same. This is because, for example, solvent replacement becomes unnecessary, the radiopharmaceutical composition can be simply prepared, and cost can be reduced.

The concentration of the radiopharmaceutical composition contained in the above stock solution may be not less than 5 MBq/mL, or not less than 25 MBq/mL. Further, the concentration may be not more than 5 GBq/mL, or not more than 500 MBq/mL.

The radiopharmaceutical composition may be stored for a storage period of not less than 30 minutes, not less than 1 hour, or not less than 3 hours. The radiopharmaceutical composition may be stored for a storage period of not more than 24 hours, not more than 18 hours, or not more than 12 hours. The radiopharmaceutical composition is stored at a storage temperature of preferably not less than 1°C, and more preferably not less than 15°C. Further, the storage temperature of the intermediate is preferably not more than 30°C, and more preferably not more than 25°C.

The present embodiment also encompasses the radiopharmaceutical composition stored by the storage method in accordance with the present embodiment for the radiopharmaceutical composition, and the stock solution (pharmaceutical formulation) containing the radiopharmaceutical composition after storage by the storage method. As described in Examples, the radiopharmaceutical composition stored by the storage method in accordance with the present embodiment for the radiopharmaceutical composition has a radiochemical purity with which the radiopharmaceutical composition can be used in real clinical settings.

In the conventional technique, since a storage method for the radiopharmaceutical composition was not established, extemporaneous preparation of the radiopharmaceutical composition was necessary. However, with use of the storage method in accordance with the present embodiment for the radiopharmaceutical composition, it is possible to stably store the radiopharmaceutical composition for 24 hours after preparation. Therefore, use of the storage method in accordance with the present embodiment for the radiopharmaceutical composition eliminates the need for extemporaneous preparation of the radiopharmaceutical composition. This makes it possible to transport, in a wide range, the radiopharmaceutical composition prepared. Further, since extemporaneous preparation of the radiopharmaceutical composition becomes unnecessary, personnel cost for preparation of the radiopharmaceutical composition and quality testing of the radiopharmaceutical composition can be significantly reduced. In addition, the storage method in accordance with the present embodiment for the radiopharmaceutical composition can conform to the pharmaceutical GMP.

Aspects of the present invention can also be expressed as follows:
A storage method in accordance with the present embodiment for an intermediate of a radiopharmaceutical composition includes storing the intermediate of the radiopharmaceutical composition in an acetate buffer, the intermediate being a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody), and the intermediate being stored for a storage period of not less than 24 hours.

In the storage method in accordance with the present embodiment, the acetate buffer may contain at least one type of neutral amino acid and at least one type of polysorbate.

In the storage method in accordance with the present embodiment, the anti-EGFR antibody may be cetuximab.

A preparation method in accordance with the present embodiment for a radiopharmaceutical composition includes the step of labeling, with a radionuclide that is coordinated with PCTA, the intermediate which has been stored by the storage method.

In the preparation method in accordance with the present embodiment for the radiopharmaceutical composition, the radionuclide may be ⁶⁴Cu or ²²⁵Ac.

A storage method in accordance with the present embodiment for a radiopharmaceutical composition includes storing the radiopharmaceutical composition in an acetate buffer, the radiopharmaceutical composition being labeled with a radionuclide which is coordinated with a 3,6,9,15-tetraazabicyclo[9.3.1]pentadeca-1(15),11,13-triene-3,6,9-triacetic acid (PCTA) and being a complex of the PCTA and an anti-epidermal growth factor receptor antibody (anti-EGFR antibody).

In the storage method in accordance with the present embodiment for the radiopharmaceutical composition, the radiopharmaceutical composition may be stored in the acetate buffer for not less than 30 minutes.

In the storage method in accordance with the present embodiment for the radiopharmaceutical composition, the radionuclide may be ⁶⁴Cu or ²²⁵Ac.

In the storage method in accordance with the present embodiment for the radiopharmaceutical composition, the anti-EGFR antibody may be cetuximab.

In the storage method in accordance with the present embodiment for the radiopharmaceutical composition, the acetate buffer may contain at least one type of neutral amino acid and at least one type of polysorbate.

An intermediate composition in accordance with the present embodiment is an intermediate composition that is used for preparation of a radiopharmaceutical composition, the intermediate composition including an intermediate of the radiopharmaceutical composition and a stock solution, the intermediate being a complex of a 3,6,9,15-tetraazabicyclo[9.3.1]pentadeca-1(15),11,13-triene-3,6,9-triacetic acid (PCTA) and an antibody, the stock solution containing at least one type of neutral amino acid, at least one type of polysorbate, and an acetate buffer, and the intermediate having been stored in the stock solution for not less than 24 hours.

A pharmaceutical formulation in accordance with the present embodiment includes a radiopharmaceutical composition and a stock solution, the radiopharmaceutical composition being labeled with a radionuclide which is coordinated with a 3,6,9,15-tetraazabicyclo[9.3.1]pentadeca-1(15),11,13-triene-3,6,9-triacetic acid (PCTA), the radionuclide being a complex of the PCTA and an antibody, the stock solution containing at least one type of neutral amino acid, at least one type of polysorbate, and an acetate buffer, and the radiopharmaceutical composition being obtained by labeling, with the radionuclide that is coordinated with the PCTA, the complex which has been stored in the stock solution for not less than 24 hours.

In the pharmaceutical formulation in accordance with the present embodiment, the radionuclide may be ⁶⁴Cu or ²²⁵Ac.

The following description will more specifically discuss an embodiment of the present invention with reference to Examples. The present invention is, of course, not limited to the Examples below and particulars can have various aspects. Further, the present invention is not limited to the above-described embodiments, but can be variously altered by a person skilled in the art within the scope of the claims. An embodiment derived from a proper combination of technical means disclosed in respective different embodiments is also encompassed in the technical scope of the present invention. Moreover, all the literatures described herein are hereby incorporated by reference.

### Examples

In the following Examples, "%" represents "% by mass" unless stated otherwise.

### [Materials and Methods]

### (Synthesis method for ⁶⁴Cu-PCTA-cetuximab)

Cetuximab, which is an EGFR antibody, was obtained by a well-known method. Specifically, a DNA strand encoding cetuximab, which was obtained with use of the gene sequence of cetuximab, was transfected into Chinese hamster ovary (CHO) cells. Cetuximab thus synthesized was purified.

According to the description in Nucl Med Biol 43 (11): 685-691, ⁶⁴Cu was prepared with use of a cyclotron, and purified. In order to form a coordination with ⁶⁴Cu and form a bond with cetuximab, 3,6,9,15-Tetraazabicyclo[9.3.1]pentadeca-1(15),11,13-triene-4-S-(4-isothiocyanatobenzyl)-3,6,9-triacetic acid (p-SCN-Bn-PCTA, Macrocyclics, Plano, TX, USA) was used. Sterilized water used was water of pharmaceutical grade, while the other chemicals used were chemicals of chemical grade.

Formation of a complex of cetuximab and PCTA and ⁶⁴Cu labeling were carried out by methods which were described in the following literatures and to which several changes were made: Cancer Sci 103 (3): 600-605; PLoS One 8 (4): e61230; and PLoS One 10 (4): e0123761. Briefly, a borate buffer (0.05 M, pH 8.5) solution (2 mg/mL) of cetuximab was prepared by replacing the buffer with use of a Vivaspin ultrafiltration device (Sartorius, Aubagne, France). The borate buffer of cetuximab was mixed with a dimethyl sulfoxide solution (0.42 mg/mL) of PCTA, at a ratio of 5:1 (PCTA: cetuximab, molar ratio). Then, a reaction was carried out at 37°C for 16 hours, so that the PCTA-cetuximab was prepared. The PCTA-cetuximab thus prepared was subjected to solution replacement so as to be 2 mg/mL. The solution replacement was carried out with use of each of three kinds of stock solutions, which are shown in Table 1, by an ultrafiltration centrifugal concentrator tube.

**[Table 1]**

| Stock solution | |
|---|---|
| Physiological saline | Comparative Example |
| 0.1 M acetate buffer (pH 6.0) | Example 1 |
| 0.1 M acetate buffer (pH 6.0) containing 100 mM glycine and 76.3 µM of polysorbate 80 (pH 6.0) | Example 2 |

With respect to each of PCTA-cetuximab solutions each having been subjected to replacement by the stock solution shown in Table 1, ⁶⁴CuCl₂ dissolved in the acetate buffer (0.1 M, pH 6.0) was added and mixed at a ratio of 3:1 (⁶⁴CuCl₂: PCTA-cetuximab, vol:vol). Then, a reaction was carried out at 40°C for 1 hour, so that ⁶⁴Cu-PCTA-cetuximab was prepared.

### [Analysis method for radiochemical purity of ⁶⁴Cu-PCTA-cetuximab]

The radiochemical purity of the ⁶⁴Cu-PCTA-cetuximab thus prepared was analyzed with use of a radio-thin layer chromatography (Radio TLC). A silica gel (silica gel 60 plate, Merck, Whitehouse Station, NJ, USA) was used as a carrier and 80% methanol (methanol: water = 80:20 (vol/vol)) was used as a mobile phase. In order to detect free ⁶⁴Cu that was not coordinated with PCTA-cetuximab, 5 pL of 25 mM EDTA was added to 5 pL of a ⁶⁴Cu-PCTA-cetuximab analysis sample. Then, analysis was carried out with use of the Radio TLC. For comparison, a 0.1 M acetate buffer (pH 6.0) solution of ⁶⁴Cu was similarly processed, and a resultant solution was used as a reference standard sample of ⁶⁴Cu. Such samples for analysis were each dripped in drops, the volume of each of which was 1 pL, onto a silica gel thin-layer plate, and were developed in the mobile phase. Then, the radiochemical purity of a TLC-plate was detected with use of an imaging analyzer (FLA7000, GE Healthcare Life Science, Marlborough, MA, USA), and respective ratios of ⁶⁴Cu-PCTA-cetuximab and ⁶⁴Cu were calculated. Relative radioactivity was calculated as a ratio of intact ⁶⁴Cu-PCTA-cetuximab relative to total radioactivity. A radiochemical purity of not less than 95% was set as a target specification criterion for the ⁶⁴Cu-PCTA-cetuximab.

### [Evaluation method for binding properties of ⁶⁴Cu-PCTA-cetuximab to cetuximab-expressing cells]

For evaluation of cell-binding properties of ⁶⁴Cu-PCTA-cetuximab, HCT116 cells (human colon cancer cells; CCL-247; American Type Cell Collection) were used. HCT116 cells were diluted in a phosphate buffer containing 1% bovine serum albumin (manufactured by Sigma) and caused to react with ⁶⁴Cu-PCTA-cetuximab for 1 hour on ice. After washing the cells, the radioactivity was measured with a gamma counter (1480 Automatic gamma counter Wizard 3, manufactured by Parkin Elmer) and the cell-binding properties of the ⁶⁴Cu-PCTA-cetuximab were evaluated. The immunobinding activity was obtained by the method of Lindmo et al. (J Immunol Methods. 1984; 72: 77-89). Also, in a competitive inhibition assay, HCT116 cells were incubated, for 1 hour on ice, with ⁶⁴Cu-PCTA-cetuximab and unlabeled cetuximab at various concentrations. After washing, the cell-binding properties of the ⁶⁴Cu-PCTA-cetuximab were evaluated as described above. On the basis of the result of the competitive inhibition assay, a dissociation constant was determined.

### [Statistical analysis]

Data on the evaluation of the cell-binding properties were expressed mean values with corresponding standard deviations. P-values were calculated by analysis of variance, and comparison of multiple groups was carried out. P values less than 0.05 were considered to be statistically significant.

### (Experiment 1. Effect of stock solution on labeling yield of ⁶⁴Cu-PCTA-cetuximab)

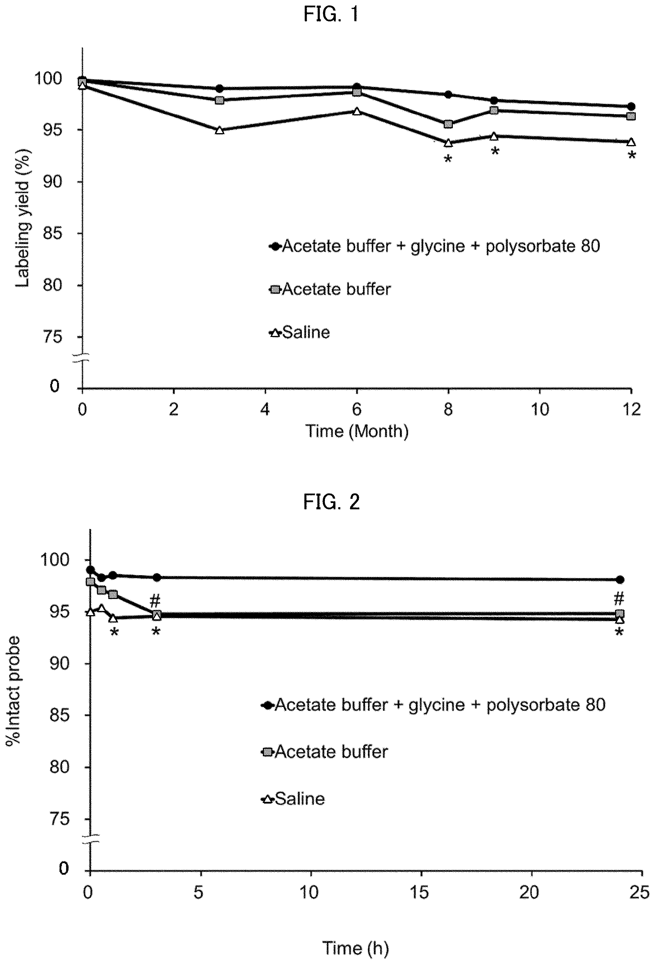

With use of PCTA-cetuximab prepared with use of the three kinds of stock solutions described in Table 1, ⁶⁴Cu-PCTA-cetuximab (⁶⁴Cu, 37 MBq, 20 pg of PCTA-cetuximab, total volume of 40 pL) was prepared. Each of samples of the PCTA-cetuximab used for preparation of the ⁶⁴Cu-PCTA-cetuximab included a sample immediately after preparation (0 months) and samples which had been stored in a refrigerator at 4°C for 3, 6, 8, 9 and 12 months. Then, the stabilizing effect of each of the three kinds of stock solutions on the PCTA-cetuximab was evaluated by comparison and study of the radiochemical purity (labeling yield) immediately after labeling. Fig. 1 shows evaluation results.

The vertical axis in Fig. 1 represents the radiochemical purity (labeling yield, %). The horizontal axis represents the storage period (months) of the PCTA-cetuximab after preparation with use of the stock solutions. The asterisk "*" indicates a result whose radiochemical purity was less than 95%.

As shown in Fig. 1, long-term storage of the PCTA-cetuximab (triangles in Fig. 1) stored in the stock solution of a comparative example resulted in a decrease in the labeling yield. The labeling yield was below 95% which was the target specification criterion. On the other hand, in cases of the PCTA-cetuximab stored in the stock solution of Example 1 (squares in Fig. 1) and in the stock solution of Example 2 (circles in Fig. 1), the decrease in labeling yield was suppressed. In particular, the PCTA-cetuximab stored in the stock solution of Example 2 achieved a significant effect of maintaining the labeling yield of the PCTA-cetuximab.

### [Experiment 2. Effect of stock solution on stability of ⁶⁴Cu-PCTA-cetuximab after labeling]

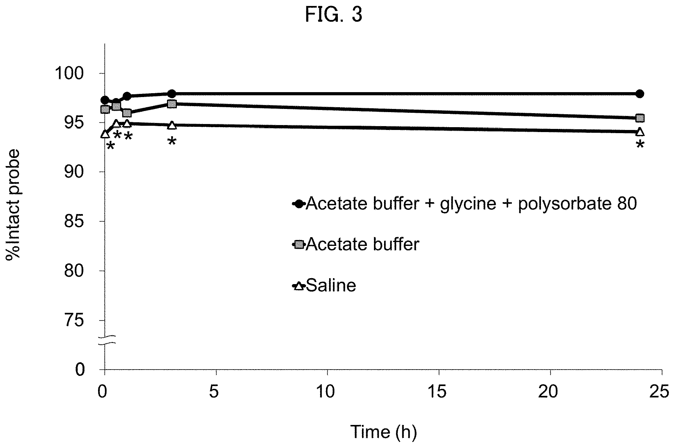

PCTA-cetuximab was prepared with use of the three kinds of stock solutions described in Table 1 above. Then, the ⁶⁴Cu-PCTA-cetuximab (⁶⁴Cu, 37 MBq, 20 pg of PCTA-cetuximab, total volume of 40 pL) was prepared with use of samples each stored at 4°C for 3 months or 12 months, respectively. The stabilizing effect of each of the three kinds of stock solutions on the ⁶⁴Cu-PCTA-cetuximab was evaluated by comparison and study of the radiochemical purity (stability) immediately after labeling and 0.5, 1, 3, and 24 hours after labeling. Figs. 2 and 3 show evaluation results.

Fig. 2 is a comparison result of the radiochemical purity (stability) after ⁶⁴Cu-labeling of the PCTA-cetuximab stored in the stock solutions for 3 months. Fig. 3 is a comparison result of the radiochemical purity (stability) after ⁶⁴Cu-labeling of the PCTA-cetuximab stored in the stock solutions for 12 months. The vertical axis in each of Figs. 2 and 3 represents the radiochemical purity (labeling yield, %). The horizontal axis represents an elapsed time after ⁶⁴Cu-labeling. The asterisk "*" and the sharp "#" indicate results whose radiochemical purity was less than 95%.

As shown in Figs. 2 and 3, for the PCTA-cetuximab (triangles in Figs. 2 and 3) stored in the stock solution of the comparison example, the radiochemical purity was below 95%, which was the target specification criterion, at the time point immediately after labeling. On the other hand, in the cases of the PCTA-cetuximab stored in the stock solution of Example 1 (squares in Figs. 2 and 3) and in the stock solution of Example 2 (circles in Figs. 2 and 3), the radiochemical purity exceeded 95%, which was the target specification criterion, at the time point immediately after labeling.

As shown in Fig. 2, the PCTA-cetuximab stored in the stock solution of Example 1 had a radiochemical purity of less than 95% after 3 hours from labeling. On the other hand, as shown in Figs. 2 and 3, the PCTA-cetuximab stored in the stock solution of Example 2 kept exhibiting the effect of maintaining the radiochemical purity for a period of not more than 24 hours from labeling, even in a case where the PCTA-cetuximab was stored for 3 months or 12 months.

Results of experiments 1 and 2 suggested, for example, the following: it is possible to transport an intermediate that is stored in the stock solution of any of Examples and to prepare a radiopharmaceutical composition from the intermediate at a destination where the intermediate has been transported. Further, it was found that the radiopharmaceutical composition can be stably stored for 24 hours by storing the radiopharmaceutical composition in the stock solution of any of Examples after preparation of the radiopharmaceutical composition.

### (Experiment 3. Effect of stock solution on cell-binding properties of ⁶⁴Cu-PCTA-cetuximab)

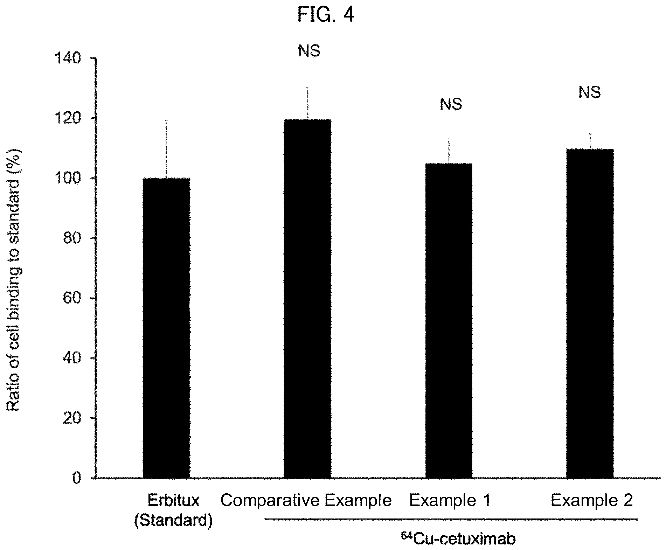

With use of the PCTA-cetuximab which had been stored in the three kinds of stock solutions described in Table 1 above at 4°C for 12 months, ⁶⁴Cu-PCTA-cetuximab (⁶⁴Cu, 37 MBq, 20 pg of PCTA-cetuximab, total volume of 40 pL) was prepared. HCT116 cells (6.25 × 10⁵ cells) were diluted in 1 mL of a phosphate buffer containing 1% bovine serum albumin (BSA). To this diluted solution, ⁶⁴Cu-PCTA-cetuximab was added and a resultant solution was incubated on ice for 1 hour. Then, the cell-binding properties of the ⁶⁴Cu-PCTA-cetuximab were compared. In accordance with the description in Non-Patent Literature 3, a complex of a commercially available cetuximab (product name: Erbitux, Merck) and PCTA, which was labeled with ⁶⁴Cu, was used as a positive control. Fig. 4 shows results of biochemical evaluation of the stabilizing effect of each of the three kinds of stock solutions on the PCTA-cetuximab.

The vertical axis in Fig. 4 represents a ratio of the cell-binding properties on the premise that the result of the cell-binding properties of the positive control was 100%. "NS" in Fig. 4 indicates that there was no significant difference as compared with a result of the cell-binding properties of the positive control.

As shown in Fig. 4, the cell-binding properties of the ⁶⁴Cu-PCTA-cetuximab were kept equivalent to those of the positive control under all storage conditions.

### [Experiment 4. Influence of radioactivity at time of labeling on cell-binding properties of ⁶⁴Cu-PCTA-cetuximab]

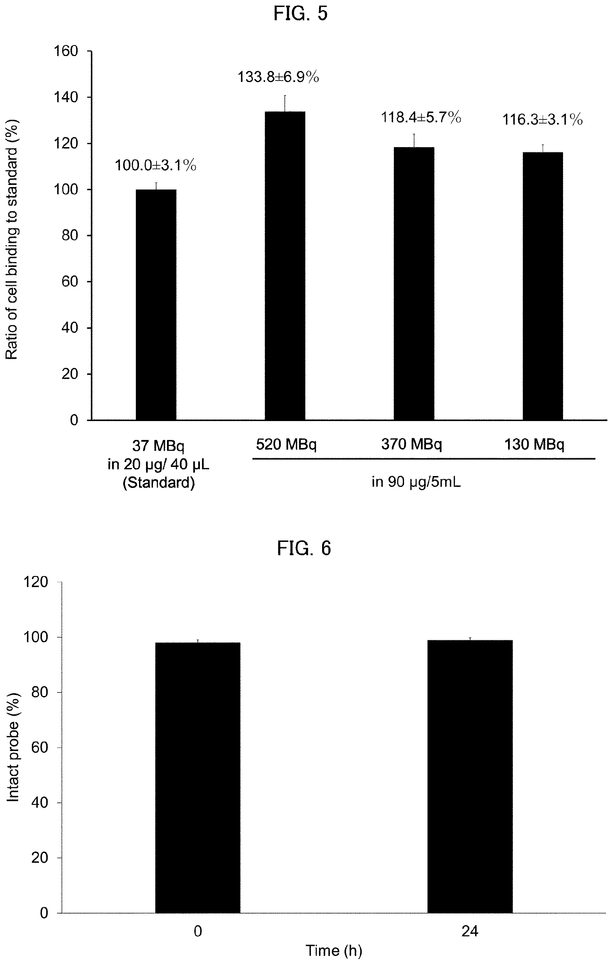

With use of the PCTA-cetuximab stored in the stock solution of Example 2, the influence of radioactivity concentrations at the time of labeling on the cell-binding properties was examined. A 0.1 M acetate buffer (pH 6.0) of ⁶⁴Cu and a PCTA-cetuximab solution were mixed at a ratio of 3:1 (⁶⁴Cu: PCTA-cetuximab; v/v). For verification, three kinds of solutions having radioactivity concentrations of 520 MBq, 370 MBq, and 130 MBq were each used as the 0.1 M acetate buffer of ⁶⁴Cu. A reaction was carried out at 40°C for 1 hour, so that ⁶⁴Cu-PCTA-cetuximab was prepared (90 pg of PCTA-cetuximab, total volume of 5 mL). Then, the cell-binding properties of the ⁶⁴Cu-PCTA-cetuximab were evaluated under the same conditions as Experiment 3. The ⁶⁴Cu-PCTA-cetuximab (⁶⁴Cu, 37MBq, 20 pg of PCTA-cetuximab, total volume of 40 pL) evaluated in Experiment 3 was used as an object to be compared. Fig. 5 shows comparison results.

The vertical axis in Fig. 5 represents a ratio of the cell-binding properties on the premise that the result of evaluation of the cell-binding properties of the ⁶⁴Cu-PCTA-cetuximab in Experiment 3 was 100%.

As shown in Fig. 5, no influence on the cell-binding properties was observed in a case where the radioactivity concentration was 520 MBq (90 pg of PCTA-cetuximab, total volume of 5 mL). On the basis of this result, it was confirmed that ⁶⁴Cu-PCTA-cetuximab could be stably prepared in volume and concentration which allow the ⁶⁴Cu-PCTA-cetuximab to be used for diagnosis in real clinical settings.

### (Experiment 5. Effect of stock solution on stability of ²²⁵Ac-PCTA-cetuximab after labeling)

PCTA-cetuximab stored in the stock solution of Example 2 was used to investigate labeling with ²²⁵Ac and stability after the labeling. With respect to the PCTA-cetuximab solution, a 0.1 M acetate buffer (pH 6.0) of ²²⁵Ac was added and mixed at a ratio of 3:1 (²²⁵Ac:PCTA-cetuximab; v/v). Then, a reaction was carried out at 40°C for 1 hour, so that ²²⁵Ac-PCTA-cetuximab was prepared. Fig. 6 shows evaluation results of the ²²⁵Ac-PCTA-cetuximab.

A bar graph on the left side of Fig. 6 shows the radiochemical purity immediately after labeling with ²²⁵Ac. A bar graph on the right side of Fig. 6 shows the radiochemical purity after 24 hours from the labeling with ²²⁵Ac.

As shown in Fig. 6, the radiochemical purity immediately after ²²⁵Ac-labeling exceeded 95%, which was the target specification criterion. Further, it was found that the radiochemical purity was maintained up to 24 hours after the ²²⁵Ac-labeling. On the basis of the above results, it was found that the present stock solution is applicable to another metallic radionuclide, such as ²²⁵Ac, other than ⁶⁴Cu.

DOTA (1,4,7,10-tetraazacyclododecane-1,4,7,10-tetraacetic acid) is widely used as a ligand in a case where cetuximab is labeled with ²²⁵Ac. In order to coordinate ²²⁵Ac with DOTA, a high pH and a high temperature are required.

On the other hand, when ²²⁵Ac-PCTA-cetuximab was prepared, it was possible to efficiently coordinate ²²⁵Ac with PCTA under mild conditions (40°C, pH 6.0). Further, the radiochemical purity was as high as not less than 95%, despite such coordination under the mild conditions.

### Industrial Applicability

An embodiment of the present invention can be used for long-term storage of an intermediate of a radiopharmaceutical composition and long-term storage of the radiopharmaceutical composition. Therefore, the present invention makes it possible to stably and efficiently produce the radiopharmaceutical composition.

## Claims

1. A storage method, comprising storing an intermediate of a radiopharmaceutical composition in an acetate buffer,
the intermediate being a complex of a 3,6,9,15-tetraazabicyclo[9.3.1]pentadeca-1(15),11,13-triene-3,6,9-triacetic acid (PCTA) and an anti-epidermal growth factor receptor antibody (anti-EGFR antibody), and
the intermediate being stored for a storage period of not less than 24 hours.

2. The storage method according to claim 1, wherein the acetate buffer contains at least one type of neutral amino acid and at least one type of polysorbate.

3. The storage method according to claim 1 or 2, wherein the anti-EGFR antibody is cetuximab.

4. A preparation method of a radiopharmaceutical composition, comprising the step of labeling, with a radionuclide that is coordinated with PCTA, the intermediate which has been stored by the storage method according to any one of claims 1 to 3.

5. The preparation method according to claim 4, wherein the radionuclide is ⁶⁴Cu or ²²⁵Ac.

6. A storage method, comprising storing a radiopharmaceutical composition in an acetate buffer,
the radiopharmaceutical composition being labeled with a radionuclide which is coordinated with a 3,6,9,15-tetraazabicyclo[9.3. 1]pentadeca-1(15),11,13-triene-3,6,9-triacetic acid (PCTA) and being a complex of the PCTA and an anti-epidermal growth factor receptor antibody (anti-EGFR antibody).

7. The storage method according to claim 6, wherein the radiopharmaceutical composition is stored in the acetate buffer for not less than 30 minutes.

8. The storage method according to claim 6 or 7, wherein the radionuclide is ⁶⁴Cu or ²²⁵Ac.

9. The storage method according to any one of claims 6 to 8, wherein the anti-EGFR antibody is cetuximab.

10. The storage method according to any one of claims 6 to 9, wherein the acetate buffer contains at least one type of neutral amino acid and at least one type of polysorbate.

11. An intermediate composition that is used for preparation of a radiopharmaceutical composition,
the intermediate composition comprising an intermediate of the radiopharmaceutical composition and a stock solution,
the intermediate being a complex of a 3,6,9,15-tetraazabicyclo[9.3.1]pentadeca-1(15),11,13-triene-3,6,9-triacetic acid (PCTA) and an antibody,
the stock solution containing at least one type of neutral amino acid, at least one type of polysorbate, and an acetate buffer, and
the intermediate having been stored in the stock solution for not less than 24 hours.

12. A pharmaceutical formulation, comprising a radiopharmaceutical composition and a stock solution,
the radiopharmaceutical composition being labeled with a radionuclide which is coordinated with a 3,6,9,15-tetraazabicyclo[9.3.1]pentadeca-1(15),11,13-triene-3,6,9-triacetic acid (PCTA), the radionuclide being a complex of the PCTA and an antibody,
the stock solution containing at least one type of neutral amino acid, at least one type of polysorbate, and an acetate buffer, and
the radiopharmaceutical composition being obtained by labeling, with the radionuclide that is coordinated with the PCTA, the complex which has been stored in the stock solution for not less than 24 hours.

13. The pharmaceutical formulation according to claim 12, wherein the radionuclide is ⁶⁴Cu.

14. The pharmaceutical formulation according to claim 12, wherein the radionuclide is ²²⁵Ac.
